# EUROPÄISCHE PATENTANMELDUNG

(11) **EP 1 231 427 A2**
(43) Veröffentlichungstag der Anmeldung: **14.08.2002**
(21) Anmeldenummer: 02002950.0
(22) Anmeldetag: 11.02.2002
(51) Int. Cl.: F21S 8/00, F21V 1/14

(54) **Leuchte**

(30) Priorität: 10.02.2001 DE 20102609 U
(71) Anmelder: Baur, Albert, 86845 Grossaitingen (DE)
(72) Erfinder: Baur, Albert, 86845 Grossaitingen (DE)
(74) Vertreter: Fiener, Josef

(57) **Zusammenfassung**

Zur verbesserten Variabilität einer Leuchte (1), bestehend aus Lichtquelle (2), Gestell (3) und Leuchtenschirm (4), wird vorgeschlagen, daß zumindest ein schalenförmiges Element (5, 6, 7, 20, 30, 105, 106, 107, 205, 206, 207, 305, 306, 307) und zumindest ein Beschwerungsgewicht (8, 108, 208, 308) über der Lichtquelle (2) anordenbar sind.

## Beschreibung

Die Erfindung bezieht sich auf eine Leuchte, bestehend aus einer Lichtquelle, einem Gestell und Leuchtenschirm.

Leuchtenschirme dienen dazu, das von der Lichtquelle ausgestrahlte Licht zu filtern und zu verteilen. So dient beispielsweise der Schirm einer über einem Esstisch angebrachten Deckenleuchte dazu, das Licht auf die Tischplatte zu richten. Im Gegensatz hierzu dient der Schirm einer Tischleuchte in der Regel dazu, das Licht gleichmäßig und eine Blendwirkung vermeidend nach allen Richtungen im Raum abzustrahlen. Durch die Wahl des entsprechendend Leuchtenschirms wird so eine völlig unterschiedliche Wirkung erzielt. Die bekannten Leuchtenschirme sind in der Regel fest auf einem Gestell, welches die Lichtquelle enthält, montiert. Sollte der Wunsch nach einer anderen Lichtart, z. B. helleres Licht oder gedämpfteres Licht, bestehen, so bedeutet dies in aller Regel, daß eine neue Leuchte beschafft und gegen die alte ausgetauscht werden muß.

Es ist auch bekannt, austauschbare Leuchtenschirme zur Verfügung zu stellen, so daß nur der Schirm abgenommen und gegen einen anderen ausgetauscht werden kann. So kann dann beispielsweise ein Papierschirm gegen einen Stoffschirm ausgetauscht werden oder ein farbiger Schirm aufgesetzt werden.

Nachteil dieser bekannten Schirme für Leuchten ist jedoch, daß die Variabilität äußerst eingeschränkt ist. Auch bei bestehender Austauschmöglichkeit ist die Flexibilität nur unzureichend. Zum einen nehmen derartige Leuchtenschirme relativ viel Lagerplatz ein, da sie zumeist nicht faltbar sind. Zum anderen sind herkömmliche Schirme in aller Regel auch sehr kostenintensiv. Als Folge dessen begnügen sich die meisten Menschen mit einer einzigen Leuchte mit einem Schirm, die dann gegebenenfalls gegen eine neue Leuchte komplett ausgetauscht wird.

Aufgabe der vorliegenden Erfindung ist es daher, die beschriebenen Nachteile zu vermeiden und eine Leuchte zur Verfügung zu stellen, die für die Schirmwahl eine gegenüber dem Stand der Technik größere Variabilität ermöglicht.

Diese Aufgabe wird gelöst durch eine Leuchte nach Anspruch 1. Vorteilhafte Ausgestaltungen sind Gegenstand der abhängigen Ansprüche.

Der große Vorteil der erfindungsgemäßen Leuchte liegt darin, daß ihr Leuchtenschirm nahezu beliebig anordenbar und veränderbar ist. Hiefür weist die erfindungsgemäße Leuchte neben einer Lichtquelle und einem Gestell zumindest ein schalenförmiges Element und zumindest ein Beschwerungsgewicht auf, die über der Lichtquelle anordenbar sind. Vorzugsweise ist vorgesehen, daß mehr als ein schalenförmiges Element vorgesehen ist, insbesondere zwei bis fünf. Die schalenförmigen Elemente können daher in ihrer Dreh- und Schwenklage beliebig angeordnet werden und mit dem Beschwerungsgewicht in dieser Lage fixiert werden. Auf diese Art und Weise lassen sich bereits mit zwei der beschriebenen schalenförmigen Elemente eine große Anzahl an Schirmgestaltungsmöglichkeiten verwirklichen. Durch den Einsatz von verschiedenen schalenförmigen Elementen erhöht sich die beschriebene Variabilität noch weiter. Die schalenförmigen Elemente können die Form von Kugelsegmenten oder Ellipsensegmenten einnehmen oder auch die Form von Blüten- oder Blumenblättern oder geometreischen Formen. So ist beispielsweise vorgesehen, daß die schalenförmigen Elemente etwa die Form von einer hohlen Halbkugel besitzen. Weiterhin ist bevorzugt vorgesehen, daß die schalenförmigen Elemente die Form eines Ellipsensegmentes haben, beispielsweise eines Achtel- oder Viertelsegmentes. Dabei können schalenförmige Elemente gleicher Größe als auch unterschiedlicher Größe miteinander beliebig kombiniert werden. Außerdem ist möglich, daß die schalenförmigen Elemente nicht nur gerundete Formen aufweisen, sondern auch abgekantet oder eckig gestaltet sein können. Es ist ferner vorgesehen, daß die schalenförmigen Elemente farbig gestaltet sein können. Dabei sind nicht nur einfarbige, sondern auch bunte oder mit beliebigen Motiven versehene schalenförmige Elemente möglich. Außerdem ist vorgesehen, daß auch verschiedenfarbige schalenförmige Elemente in einer Leuchte kombinierbar sind. Durch diese beschriebenen Variationsmöglichkeiten der schalenförmigen Elemente ist eine nahezu beliebige, individuelle Gestaltung der erfindungsgemäßen Leuchte gegeben.

Ein weiterer Vorteil ist, daß die schalenförmigen Elemente relativ platzsparend zu lagern sind, so daß es dem Verbraucher möglich ist, mehrere, auch verschiedene schalenförmige Elemente anzuschaffen und zu Hause aufzubewahren, so daß die Schirmform der Leuchte jederzeit abgewandelt werden kann.

Als Materialien für die schalenförmigen Elemente kommen alle Werkstoffe in Frage, die für die Ausrichtung eines Lichtstrahles und/oder dessen Filterung geeignet sind. Vorzugsweise ist vorgesehen, daß die schalenförmigen Elemente aus Glas oder Kunststoff bestehen. Ein weiterer Vorteil der erfindungsgemäßen Leuchte liegt darin, daß auch aufgrund der Materialwahl, die schalenförmigen Elemente relativ kostengünstig herstellbar sind, da bei einer relativ geringen Anzahl an Formen eine größe Anzahl an Variationsmöglichkeiten besteht, ohne daß für jede Gestaltungsmöglichkeit gesonderte Schirme angefertigt werden müßten.

Das Beschwerungsgewicht ist so gestaltet, daß es die schalenförmigen Elemente in einer gewählten Position fixiert. Hierfür muß das Beschwerungsgewicht eine gewisse Masse aufweisen. Vorzugsweise ist vorgesehen, daß das Beschwerungsgewicht kugelförmig gestaltet ist, obwohl andere Form ebenfalls möglich sind. Als Materialien für das Beschwerungsgewicht können sowohl lichtdurchlässige als auch lichtundurchlässige verwendet werden. So kann das Beschwerungsgewicht beispielsweise aus Metall, z. B. aus Edelstahl, bestehen aber auch aus Keramik, Glas oder Naturstein. Wird für die Herstellung des Beschwerungsgewichtes Glas eingesetzt, so kann dieses zudem geschliffen sein, so daß die Lichtstrahlen von der Lichtquelle an den Schliffkanten gebrochen werden, was weitere Lichtstreueffekte bewirkt. Vorzugsweise ist vorgesehen, daß die schalenförmigen Elemente und das Beschwerungsgewicht aus unterschiedlichen Materialien gefertigt sind, da hierdurch besondere optische Effekte erzielbar sind. Auch für das Beschwerungsgewicht gilt, daß es variiert werden kann und daß auch mehrere in einer Lampe zugleich angeordnet werden können. Dadurch bieten sich dem Verbraucher weitere Variationsmöglichkeiten; so kann er z. B. mit einem lichtundurchlässigen und einem lichtdurchlässigen Beschwerungsgewicht das Leuchtbild variieren.

Als Lichtquelle können die bekannten Glühbirnen genauso wie Halogenlampen verwendet werden. Es kann weiterhin vorgesehen sein, daß zusätzlich eine drehbare Filterscheibe, insbesondere eine Farbfilterscheibe über der Lichtquelle angeordnet ist, die bevorzugt über eine Fernbedienung in ihrer Farbe oder Intensität einstellbar ("dimmbar") ist. Auf diese Art und Weise werden weitere ausgezeichnete optische Möglichkeiten erzielt.

Als Gestell ist jegliches Untergestell denkbar, das so gestaltet ist, daß die schalenförmigen Elemente über einer Lichtquelle anordenbar sind, bevorzugt auf einer wannenförmigen Auflage, in die auch Duftöl eingefüllt werden kann. Beispielhaft seien hier säulenförmige Leuchten genannt, auf deren Oberseite die schalenförmigen Elemente gruppierbar sind. Weiterhin seien Gestelle erwähnt, auf deren Oberseite eine lichtdurchlässige Scheibe, beispielsweise eine konkav gewölbte Glas- oder Kunststoffscheibe aufliegt, auf der wiederum die schalenförmigen Elemente gruppierbar sind.

Nachstehend werden einige Ausführungen der Erfindung anhand der Zeichnungen näher erläutert und beschrieben. Es zeigen:
- Fig. 1:: eine perspektivische, schematische Darstellung eines Ausführungsbeispiels einer erfindungsgemäßen Leuchte;
- Fig. 2:: ein Ausführungsbeispiel für ein schalenförmiges Element in Seitenansicht (A) bzw. Draufsicht (B);
- Fig. 3:: ein weiteres Ausführungsbeispiel eines schalenförmigen Elementes in Seitenansicht;
- Fig. 4:: ein Ausführungsbeispiel der Anordnung meherer schalenförmiger Elemente in Vorderansicht;
- Fig. 5:: das Ausführungsbeispiel der Fig. 4 in Seitenansicht;
- Fig. 6:: das Ausführungsbeispiel der Fig. 4 in Draufsicht;
- Fig. 7:: ein weiteres Ausführungsbeispiel für die Anordnung der schalenförmigen Elemente in Draufsicht; und
- Fig. 8:: ein weiteres Ausführungsbeispiel für die Anordnung der schalenförmigen Elemente in Draufsicht.

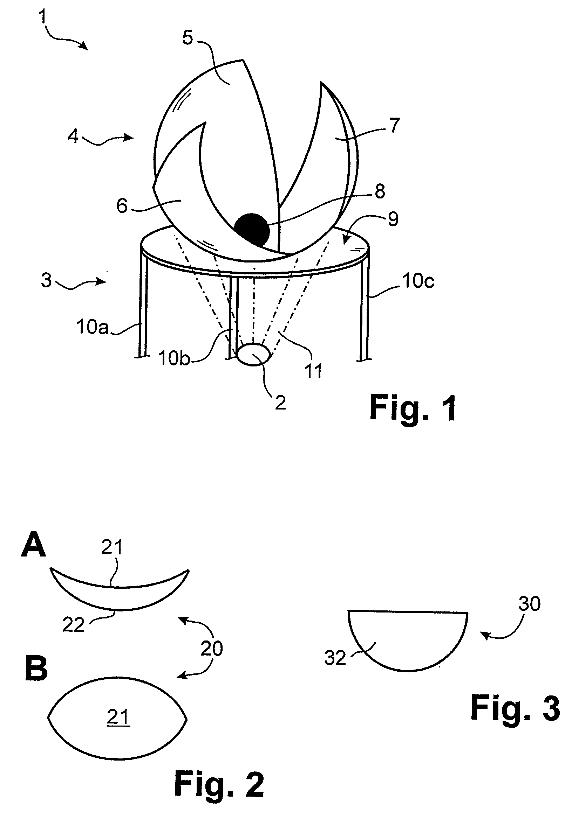

In Fig. 1 ist eine Leuchte 1, bestehend aus einer Lichtquelle 2, einem Gestell 3 und einem Leuchtenschirm 4, dargestellt. Im Ausführungsbeispiel der Fig. 1 sind drei schalenförmige Elemente 5, 6 und 7 mit einem Beschwerungsgewicht 8 zum Leuchtenschirm 4 angeordnet. Die schalenförmigen Elemente 5, 6 und 7 weisen eine Segmentform auf, im vorliegenden Ausführungsbeispiel die eines Ellipsensegmentes. Die schalenförmigen Elemente 5, 6 und 7 können in Form, Farbe und Größe sowohl identisch als auch voneinander verschieden sein. In Fig. 1 ist weiterhin ein Beschwerungsgewicht 8 dargestellt, das mittels seiner Masse dazu dient, die schalenförmigen Elemente 5, 6 und 7 in ihrer Anordnung zu fixieren. Die schalenförmigen Elemente 5, 6 und 7 können dabei in den Raumachsen verschoben, gedreht, geschwenkt oder gekippt werden, um so zueinander in Art einer Blüte angeordnet werden. So können diese beispielsweise unmittelbar hintereinander angeordnet werden aber auch in einem gleichmäßigen oder ungleichmäßigen Winkel zueinander stehen; im Ausführungsbeispiel der Fig. 1 sind es beispielsweise annähernd 120°. Weiterhin ist deren Anzahl beliebig wählbar, im Ausführungsbeispiel der Fig. 1 sind drei schalenförmige Elemente 5, 6 und 7 angeordnet. Bereits durch die Verwendung eines einzigen schalenförmigen Elementes 5, 6 oder 7 und mittels eines einzigen Beschwerungsgewichtes 8 sind etliche Variationen möglich. So kann ein einzelnes schalenförmiges Element 5, 6 oder 7 beispielsweise in aufrechter Position, ähnlich wie in Fig. 1 dargestellt, positioniert werden, oder aber es kann das Beschwerungsgewicht 8 mehr oder weniger in der Mitte eines schalenförmigen Elementes 5, 6 oder 7 plaziert werden. Weiterhin kann das schalenförmige Element 5, 6 oder 7 so angeordnet werden, daß seine konkave oder aber auch seine konvexe Seite dem Betrachter zugewandt ist. Es lassen sich folglich bereits durch den Einsatz eines einzigen schalenförmigen Elementes 5, 6 oder 7 und eines Beschwerungsgewichtes 8 zahlreiche Variationen erzielen.

Diese vielfältigen Anordnungsmöglichkeiten werden anhand der Erläuterung weiterer Figuren noch deutlicher. Das Gestell 3 der Leuchte 1 in Fig. 1 ist lediglich schematisch dargestellt. Im beschriebenen Ausführungsbeispiel besteht es aus einer lichtdurchlässigen Auflage 9, beispielsweise aus Glas und diese Auflage 9 abstützende Füße 10a, b, c. Im Gebrauch strahlt die Lichtquelle 2 ihr Licht durch die bevorzugt konkave Auflage 9 des Gestelles 3 und durch die schalenförmigen Elemente 5, 6 und 7 in den Raum (angedeutet mit strichpunktierten Linien 11).

Fig. 2 zeigt ein Ausführungsbeispiel eines schalenförmigen Elementes 20 in Seitenansicht (A) und Draufsicht (B). Das schalenförmige Element 20 weist die Form eines Ellipsensegmentes auf und enthält eine konkave Seite 21 und eine konvexe Seite 22.

Fig. 3 zeigt ein weiteres Ausführungsbeispiel eines schalenförmigen Elementes 30. Es weist die Form einer halben Hohlkugel auf, so daß folglich lediglich die konvexe Seite 32 in Fig. 3 ersichtlich ist.

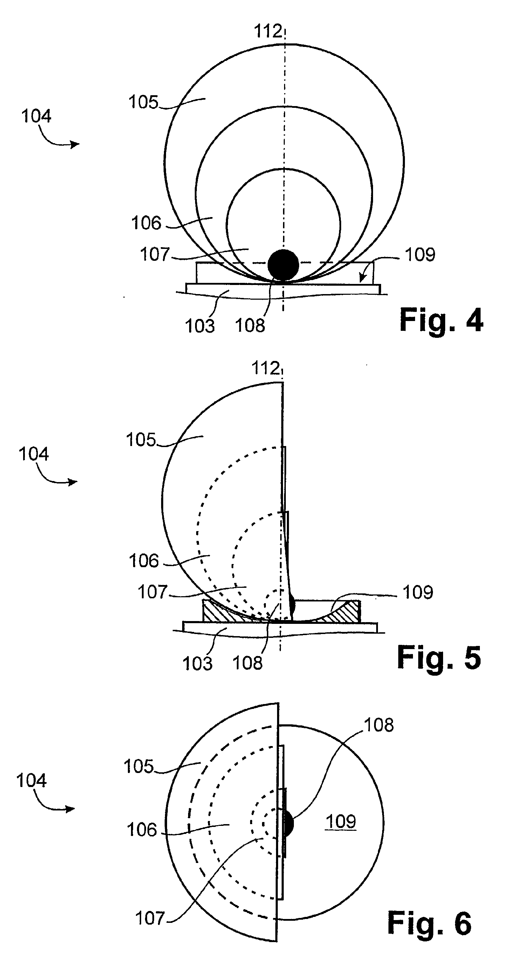

Die Fig. 4, 5 und 6 zeigen jeweils unterschiedliche Ansichten einer Schirmanordnung 104 eines weiteren Ausführungsbeispiels einer erfindungsgemäßen Leuchte. Dargestellt sind die Vorderansicht (Fig. 4), Seitenansicht (Fig. 5) und Draufsicht (Fig. 6). Die Schirmanordnung 104 besteht aus drei schalenförmigen Elementen 105, 106 und 107 unterschiedlicher Größe bzw. Durchmesser, die jedoch alle annähernd die Form einer Halbkugel aufweisen. Die schalenförmigen Elemente 105, 106 und 107 sind entsprechend ihrer Größe ineinander gestapelt. Ferner sind sie so angeordnet, daß ihre Mittelpunkte auf einer Achse 112 liegen. Weiterhin sind die schalenförmigen Elemente 105, 106 und 107 in ihrer Position durch das Beschwerungsgewicht 108 fixiert. Die schalenförmigen Elemente 105, 106 und 107 sind im Ausführungsbeispiel der Fig. 4 bis 6 auf der oberseitigen Auflage 109 eines zylinderförmigen Gestelles 103 angeordnet, wobei gemäß dem Teilschnitt in Fig. 5 die Auflage 109 konkav bzw. wannenförmig ausgebildet ist, um Duftöl aufnehmen zu können. Zudem wird durch die konkave oder ringförmige Gestaltung der Auflage 109 die Fixierung der Schalenelemente 105 - 107 gesteigert, so daß auch deren hochstehende Anordnung möglich ist. Auf die Darstellung einer Lichtquelle wurde im Ausführungsbeispiel der Figuren 4 bis 6 verzichtet.

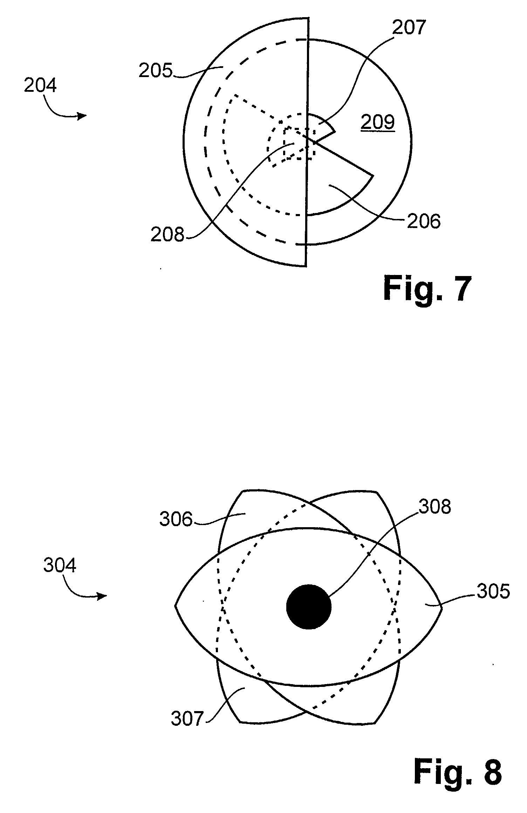

Fig. 7 zeigt eine weitere Anordnungsmöglichkeit von schalenförmigen Elementen 205, 206 und 207 auf einer Auflage 209 in Draufsicht. Für die Anordnung des Schirms 204 werden schalenförmige Elemente 205, 206 und 207 verwendet, die in den Form- und Größenverhältnissen ähnlich den schalenförmigen Elementen 105, 106 und 107 der Fig. 4 bis 6 sind. Im Ausführungsbeispiel der Fig. 7 sind die schalenförmigen Elemente 105, 106 und 107 jedoch bezüglich der Hochachse zueinander verdreht angeordnet. Auch im vorliegenden Ausführungsbeispiel dient ein Beschwerungsgewicht 208 zur Fixierung der schalenförmigen Elemente 205, 206 und 207 in der gewählten Position. Das Beschwerungsgewicht 208 weist dabei im Ausführungsbeispiel der Fig. 7 Würfelform auf.

Fig. 8 zeigt ein weiteres Ausführungsbeispiel einer erfindungsgemäßen Anordnung eines Leuchtenschirms 304 in Draufsicht. Hierfür sind drei schalenförmige Elemente 305, 306 und 307 versetzt zueinander angeordnet. Diese schalenförmigen Elemente 305, 306 und 307 weisen eine blätter- bzw. blütenartige Form ähnlich denen der Fig. 2 auf. Die Fixierung der schalenförmigen Elemente 305, 306 und 307 in ihrer Anordnung erfolgt wiederum durch ein Beschwerungsgewicht 308.

## Patentansprüche

1. Leuchte (1), bestehend aus Lichtquelle (2), Gestell (3) und Leuchtenschirm (4),
**dadurch gekennzeichnet, daß**
zumindest ein schalenförmiges Element (5, 6, 7, 20, 30, 105, 106, 107, 205, 206, 207, 305, 306, 307) und zumindest ein Beschwerungsgewicht (8, 108, 208, 308) über der Lichtquelle (2) anordenbar sind.

2. Leuchte nach Anspruch 1,
**dadurch gekennzeichnet, daß**
mehr als ein schalenförmiges Element (5, 6, 7, 20, 30, 105, 106, 107, 205, 206, 207, 305, 306, 307), insbesondere zwei bis fünf vorgesehen sind.

3. Leuchte nach Anspruch 1 oder 2,
**dadurch gekennzeichnet, daß**
das schalenförmige Element als Kugelsegment (30, 105, 106, 107, 205, 206, 207) und/oder Ellipsensegment (5, 6, 7, 20, 305, 306, 307) geformt ist.

4. Leuchte nach einem der vorhergehenden Ansprüche,
**dadurch gekennzeichnet, daß**
in Form, Größe und/oder Farbe unterschiedliche schalenförmige Elemente (5, 6, 7, 20, 30, 105, 106, 107, 205, 206, 207, 305, 306, 307) miteinander kombinierbar sind.

5. Leuchte nach einem der vorhergehenden Ansprüche,
**dadurch gekennzeichnet, daß**
das schalenförmige Element (5, 6, 7, 20, 30, 105, 106, 107, 205, 206, 207, 305, 306, 307) aus einem lichtdurchlässigen Material, insbesondere aus Glas oder Kunststoff besteht.

6. Leuchte nach einem der vorhergehenden Ansprüche,
**dadurch gekennzeichnet, daß**
das Beschwerungsgewicht (8, 108, 308) kugelförmig ist.

7. Leuchte nach einem der vorhergehenden Ansprüche,
**dadurch gekennzeichnet, daß**
das Beschwerungsgewicht (8, 108, 208, 308) aus einem lichtundurchlässigen Material, insbesondere Metall, Stein, Keramik und/oder aus einem lichtdurchlässigen Material, insbesondere Glas oder Kristall besteht.

8. Leuchte nach einem der vorhergehenden Ansprüche,
**dadurch gekennzeichnet, daß**
die schalenförmigen Elemente (5, 6, 7, 20, 30, 105, 106, 107, 205, 206, 207, 305, 306, 307) auf einer ringförmigen bzw. konkaven Auflage (9, 109, 209) aufliegen, in die insbesondere ein Duftöl einfüllbar ist.

9. Leuchte nach einem der vorangegangenen Ansprüche,
**dadurch gekennzeichnet, daß**
zwischen der Lichtquelle (2) und dem Leuchtenschirm (4, 104, 204, 304) eine drehbare Filterscheibe vorgesehen ist, insbesondere eine Farbfilterscheibe, und/oder die Lichtquelle (2) farb- bzw. intensitätsverstellbar ist, insbesondere über eine Fernbedienung.
